(19)

Europäisches
Patentamt
European
Patent Office
Office européen
des brevets

(11) **EP 2 310 873 B1**

(12) **EUROPEAN PATENT SPECIFICATION**

(45) Date of publication and mention
of the grant of the patent:
**29.05.2013 Bulletin 2013/22**

(51) Int Cl.:
***G01S 15/89*** *(2006.01)*    ***A61B 8/14*** *(2006.01)*

(21) Application number: **09786861.6**

(22) Date of filing: **07.08.2009**

(86) International application number:
**PCT/IB2009/053487**

(87) International publication number:
**WO 2010/018513 (18.02.2010 Gazette 2010/07)**

(54) **METHOD OF MESHING AND CALCULATING A VOLUME IN AN ULTRASOUND IMAGING SYSTEM**

VERFAHREN ZUR VERMASCHUNG UND BERECHNUNG EINES VOLUMENS IN EINEM
ULTRASCHALLBILDGEBUNGSSYSTEM

PROCÉDÉ DE MAILLAGE ET DE CALCUL D UN VOLUME DANS UN SYSTÈME D IMAGERIE
ULTRASONIQUE

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **12.08.2008 US 88121 P**

(43) Date of publication of application:
**20.04.2011 Bulletin 2011/16**

(73) Proprietor: **Koninklijke Philips Electronics N.V.
5621 BA Eindhoven (NL)**

(72) Inventors:
• **VION, Michael
NL-5656 AE Eindhoven (NL)**
• **SNOOK, Allen
NL-5656 AE Eindhoven (NL)**

(74) Representative: **Van Velzen, Maaike Mathilde
Philips
Intellectual Property & Standards
P.O. Box 220
5600 AE Eindhoven (NL)**

(56) References cited:
**US-A- 6 106 466**      **US-A1- 2005 240 104
US-A1- 2008 051 653**

• **MONTAGNAT J ET AL: "Surface simplex meshes
for 3D medical image segmentation" ROBOTICS
AND AUTOMATION, 2000. PROCEEDINGS. ICRA
'00. IEEE INTERNATION AL CONFERENCE ON,
PISCATAWAY, NJ, USA, IEEE, vol. 1, 24 April 2000
(2000-04-24), pages 864-870, XP002373226 ISBN:
978-0-7803-5886-7**

**Description**

FIELD OF THE INVENTION

The present invention relates to a method of meshing a volume acquired by a 3D ultrasound imaging system. The present invention also relates to a method of calculating a volume of an object imaged with such a system. The present invention also relates to a device and a computer program product related to said methods.

BACKGROUND OF THE INVENTION

There exist many applications of ultrasound imaging in the medical field. Ultrasound imaging systems are used in obstetric applications for acquiring images of the foetus in order to monitor its development. Such systems are also used in cancer diagnostic applications for determining the size of a tumor. Several parts of the body can be examined: the liver, the breast, the thyroid gland etc... Ultrasound imaging for diagnosis purposes is also used for monitoring plaque in the carotid arteries, or for detecting torn muscles.

In diagnostic applications, there is a need for measuring parts of the body. For example, there is a need for measuring the length of a bone, the volume of a liver or the gallbladder, measuring an angle between two bones etc.

Ultrasound imaging systems providing measurement functions are already known. In such known systems, a set of three dimensional (3D) image data is acquired in view of an object to be imaged by means of an ultrasound probe. Then, several views of the object are displayed to a practitioner who has to make a diagnostic.

In the known systems, the practitioner has to browse among the views offered by the system, in order to determine a main axis of the object. The practitioner draws by hand the main axis, for example using a mouse or a stylus. This main axis is used for defining a set of planes comprising slices of the object and for drawing contours of the slices.

The planes are defined perpendicularly to the main axis. Once the planes are defined, the practitioner goes from one plane to another to draw the borders of the slice of the object imaged in the current plane. Then, the system perpendicularly stacks along the mains axis, and according to a predetermined spacing, the contours drawn by the practitioner in each plane.

Then, for instance, for calculating the volume of the object, the sum of the conical frustum volumes delimited by two successive contours along the main axis is calculated.

The method used in the prior art is fastidious for the practitioner who has to manually draw the contours in each slice. In order to reach an acceptable precision of the measurement, the practitioner has typically to draw up to 15 contours. Thus, in the prior art, the measurement relies on the practitioner's experience for precisely drawing the contours in each plane as mentioned above. Moreover, the process for arriving at the volume calculation is very long, which is not compatible with the use of the ultrasound imaging means in hospitals.

Moreover, the measurement relies on the determination of the main axis, which is determined by hand. Even if the practitioner can choose several levels of zoom in the images, the determination of the main axis is subject to errors, and then, the result may be imprecise.

Document US 6 106 466, discloses a method for defining a three-dimensional surface of at least a portion of a patient's heart, based on data obtained by ultrasound imaging of the heart with a manual intervention.

Thus, there is a need for a measurement method that limits the user's intervention in an ultrasound imaging system.

SUMMARY OF THE INVENTION

According to a first aspect of the invention, there is provided a method of automatically meshing a volume in an ultrasound imaging system, said method comprising the following steps:

a) acquiring a set of 3D image data of an object;
b) selecting by a user a first surface of interest in the 3D image data, said first surface of interest comprising a first slice of the object;
c) automatically determining by the system a main axis of the object in the first surface of interest;
d) defining by the system a first set of planes of the 3D image data, these planes being not parallel with the main axis while being parallel one with respect to each other with a given distance between two successive planes along the main axis;
e) for at least two planes of the first set of planes, each comprising a respective second slice of the object, automatically drawing by the system a contour of the second slice;
f) automatically meshing by the system the volume of the object, by stacking the contours drawn in said at least two planes of the first set of planes along the main axis and by separating these planes by said given distance.

Thus, the present invention provides a method that significantly reduces the human intervention notably because the contours and the main axis are automatically determined by the system.

The main axis may be automatically determined by applying a border detection algorithm in the first surface of interest for recognizing the first slice, and by selecting a segment in the recognized slice as the main axis.

Moreover, the selected segment may be the longest segment in the recognized slice. Hence, a large number of contours may be determined, increasing the precision of the meshing.

According to a particular embodiment, the method further comprises the steps of:

- displaying at least an image of the first surface of interest;
- acquiring a user input indicating a region of the surface of interest; and
- initiating the border detection algorithm in the region indicated by the user.

Hence, the meshing process is accelerated by indicating to the algorithm where to start the contour recognition. Indeed, the practitioner usually has a solid experience of medical imaging and can rapidly identify the region where the slice of the object appears.

Step e) may comprise the following sub-steps:

e1) calculating a center of gravity of a contour drawn in one plane of the set of planes;
e2) selecting a second surface of interest in another plane of the set of plane which is adjacent to said one plane along the main axis based on the calculated center of gravity; and
e3) triggering a border detection algorithm in said second surface of interest for drawing the contour in said another plane of the set of planes.

Thus, contours may be detected in the planes without any intervention of the practitioner who uses the imaging system. The contour detected in a plane serves for determining the surface of interest in another plane.

According to another embodiment, the method further comprises the steps of:

- determining a second set of planes comprising at least two reference planes in the set of 3D data, said reference planes being not parallel one with respect to each other;
- displaying 2D images of the set of 3D image data according to said reference planes;
- selecting one reference plane for the selection of the first surface of interest in step b); and
- bringing to coincidence one plane of the set of planes with the other reference plane.

The reference planes may be displayed to a practitioner who uses the imaging system for a better comfort. These reference planes may help the practitioner to have an idea of the global shape of the object imaged.

Three reference planes may be determined, each plane being perpendicular to the others, and the second surface of interest in at least one plane of the set of planes may be designated by a point of intersection of the three reference planes. Hence, the practitioner does not have to indicate where to search for the contour, and the initiation of the contour detection process is facilitated by indicating the surface of interest in the first considered plane.

According to second aspect of the invention, a method of calculating a volume is provided.

According to a third aspect of the invention, there is provided a computer program product comprising instructions for implementing the meshing method according to at least one of the above discussed embodiments of the invention when loaded and run on computer means of an ultrasound imaging device. A computer program product comprising instructions for implementing the volume calculating method is also provided.

According to other aspects of the invention, there are provided devices for meshing a calculating a volume comprising means for implementing the method according to at least one of the above discussed aspects of the invention.

BRIEF DESCRIPTION OF THE DRAWINGS

Other features and advantages of the invention will become apparent from the following description of non-limiting exemplary embodiments, with reference to the appended drawings, in which:

- Figure 1 is a schematic illustration of an object and a slice of this object represented according to a given plane;
- Figure 2 is a schematic illustration of an object imaged according to three reference planes;
- Figure 3 is a schematic illustration of the object of figure 2 imaged according to the reference planes aligned with respect to the main axis of the object;
- Figure 4 is a schematic illustration of a meshed volume;
- Figure 5 is a flow chart depicting steps of the method, from the data acquisition to the planes alignment;
- Figure 6 is a flow chart depicting steps of the contour detection; and
- Figure 7 is a schematic illustration of an ultrasound imaging device for implementing the meshing method.

DETAILED DESCRIPTION OF THE INVENTION

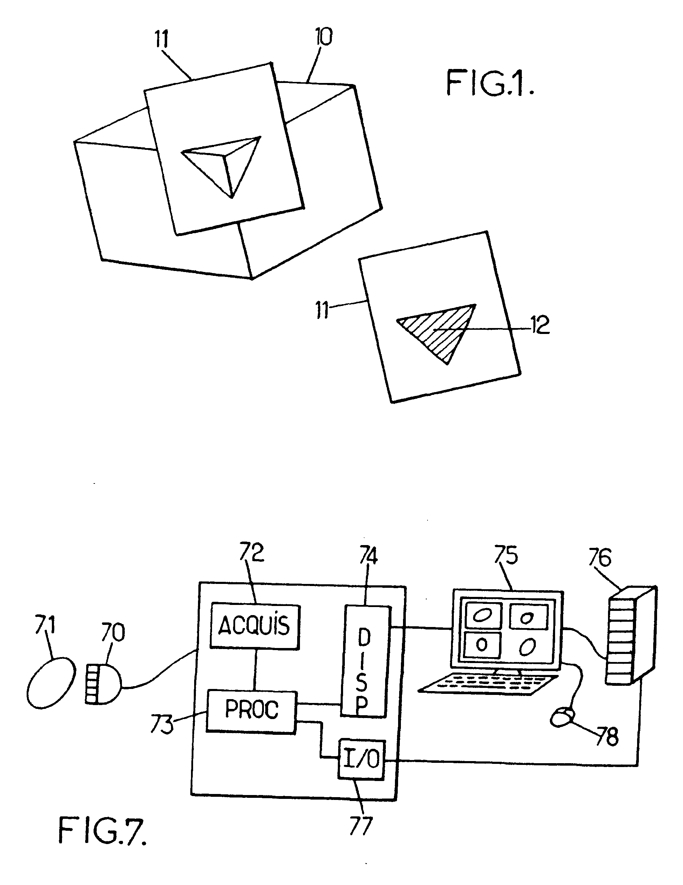

In the present description, when it is referred to a slice of an object, it is referred to a view of an intersection of the object with a given plane. Referring to Figure 1, a three dimensional (3D) object 10 and a plane 11 intersect in a region 12, visible on the plane 11, called hereinafter a slice of the object.

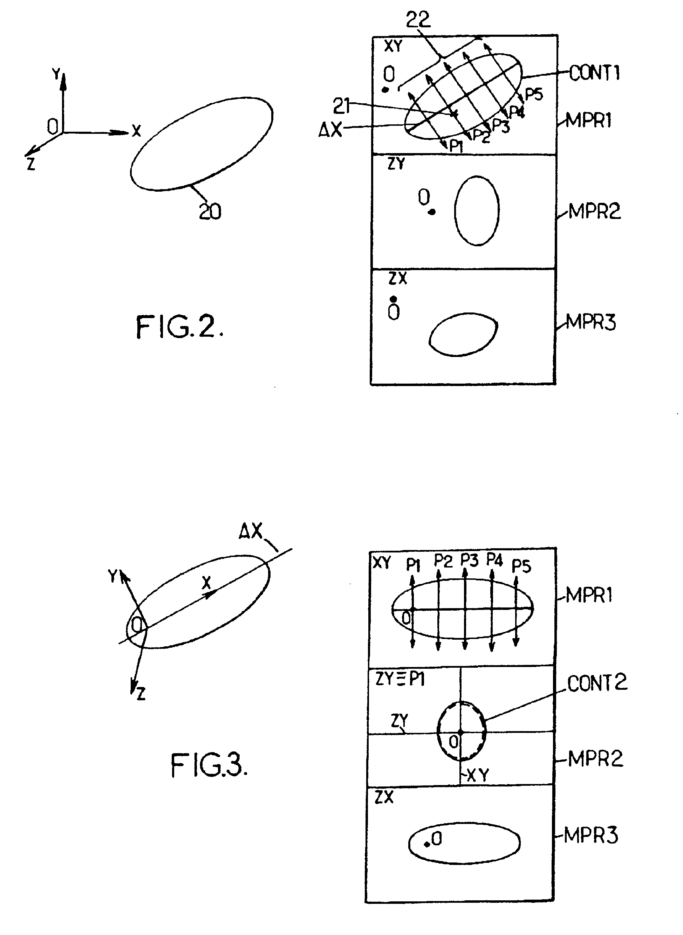

As represented in Figure 2, an object has been imaged according to three reference planes. These planes are described with reference to the orthonormal referential (O, x, y, z). Hence, the reference planes (O, x, y), (O, z, y), (O, z, x) have a given orthogonal position one relatively to the other.

In Figure 2, a view of the object 20 according to each reference plane is displayed to a user. These views are referred to as MPRs for "Multiplanar reconstruction". Each MPR (MPR1, MPR2, MPR3) shows a slice of the object 20.

The user makes a choice between these MPRs, in order to select a surface of interest in the chosen MPR. A particular choice may be the MPR that has the best resolution. Indeed, as the border detection algorithm will be implemented on the chosen MPR in order to determine the main axis, a plane with a good resolution can be preferred.

One particular MPR may correspond to a plane of acquisition. This plane of acquisition corresponds to a plane orthogonal to the beams of the ultrasound system. This type of plane usually has a good resolution.

In Figure 2, the user chooses MPR1 for launching the border detection algorithm. For that purpose, the user clicks on the MPR in the slice at the location 21 corresponding preferably to the interior of the border. The click action then causes a launch of the algorithm, and the slice's contour CONT1 is detected. For example, the border detection algorithm is a pattern recognition algorithm such as the co-called fast marching algorithm, or such as the so-called snake algorithm.

Then, a computer program launches another algorithm for determining the main axis AX. The longest segment comprised in the slice is chosen as the mains axis. As usually, the objects imaged have an ellipsoidal shape, the main axis may correspond in this case to the main axis of the ellipsoid.

Once the main axis AX is determined, it is divided into equal parts for a regular meshing. However, the parts may be of different sizes.

At each division of the main axis, a plane P1, P2, P3, P4, P5, orthogonal to the main axis is defined. These planes define a set of planes 22, one parallel to the other while being not parallel to the main axis with a given distance between two successive planes along the main axis.

Referring now to figure 3, the same object as in figure 2 is imaged, still according to the MPRs, but now, the MPRs are aligned such that plane (O, x, y) coincides with plane P1.

Once the MPRs are aligned, the computer program launches a border detection algorithm in MPR2, in a surface of interest found to be the intersection of MPR1 and MPR3 in MPR2, that is to say point O. Thus, no intervention is needed from a user of the ultrasound imaging system.

The border detection algorithm determines the contour CONT2 of the slice of MPR2.

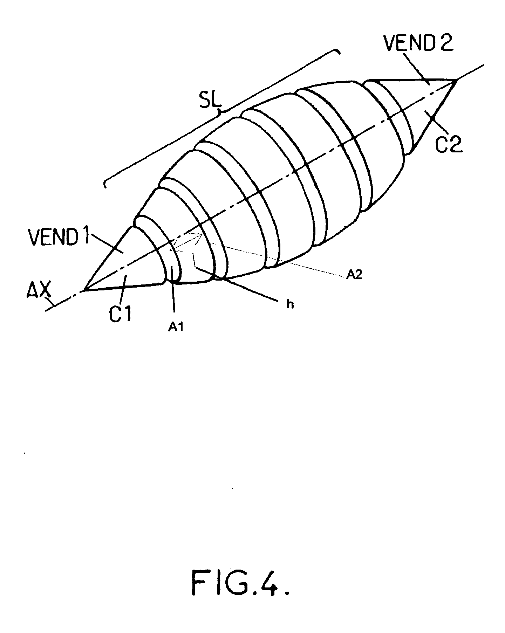

By aligning the MPRs successively with each plane P1, P2, P3, P4, P5, and by determining contours in each of said planes, the object is meshed as shown in figure 4.

The meshing of the object comprises a set of slices SL delimited by the contours detected in each plane P1, P2, P3, P4, P5, and the distance between two consecutive planes.

If the object has a peaked extremity, the main axis passes by this peaked extremity, and the method further comprises determining an end cone corresponding to the peaked extremity of the object. This enables a precise meshing of the object.

In figure 4, the meshing comprises two end cones C1, and C2. These cones are determined in order to more precisely mesh the object. Indeed, in the particular case illustrated in figure 4, the object has a peaked shape.

Then, in order to determine a volume VOL of the object, the sum of the conical frustum volumes delimited by two successive contours along the main axis is calculated. Such conical frustum volumes may be calculated according to the formula:

$$\frac{1}{3}.h.(A1 + A2 + \sqrt{A1.A2})\,,$$ wherein A1 and A2 respectively correspond to the areas of the two successive contours along the main axis and h corresponds to the distance between the two successive contours. These conical frustum volumes approximate the sub-volumes of the slices. Indeed, the contours detected may not have a circular shape, but any other shape.

The volume VOL is then calculated by summing all the conical frustum volumes, which approximate the sub-volumes of the slices, with the sub-volumes of the end cones.

The volume may also be calculated according to the Simpson's method used for cardiac volume measurements

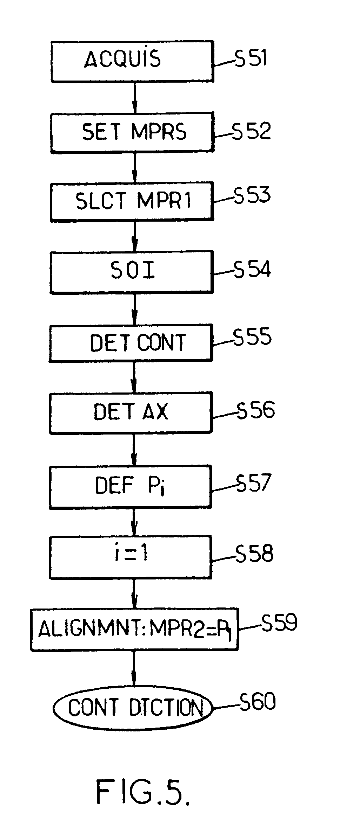

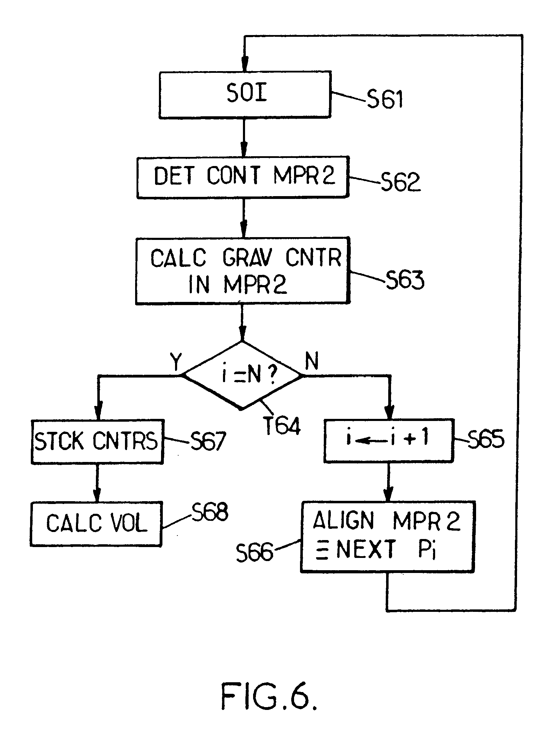

Figures 5 and 6 show flowcharts of the steps carried out in the above described method.

First, at step S51, 3D image data of an object are acquired by means of an ultrasound probe. Then, three reference planes among the 3D data are set at step S52. Each reference plane is perpendicular to the others. One of these reference planes is selected, at step S53, for the definition of the main axis in the 3D data. The images according to the three planes may be displayed simultaneously.

The user of the ultrasound imaging system, at step S54, clicks on a surface of interest in a displayed image of the reference plane that has been selected. For that purpose, the user is provided with a mouse or a stylus. Then, at step S55 the border detection algorithm is launched in the selected surface of interest in order to detect and draw the contour of the object in the reference plane. Once the contour is drawn by the detection algorithm, the main axis of the object is determined, at step S56, by selecting the longest segment in the contour.

From the main axis, a set of planes Pi with i an integer from 1 to 5 is automatically defined. Each plane of the set of planes is parallel to the others while being not parallel to the main axis. Preferably, these planes are normal to the main axis.

Then, the contour detection process is automatically initiated at step S58 by setting i to 1, that is to say by selecting plane P1.

Firstly, the reference planes are aligned such that one plane P1, P2, P3, P4, P5 of the set of planes Pi coincides with a reference plane other than the one from which the main axis was determined. The contour detection process S60 is explained in more details with reference to Figure 6.

In order to initiate the first contour detection, the intersection of the reference planes is determined. This intersection serves as a "seed" (i.e. starting point) for determining the surface of interest in the first plane P1 considered in the set of planes Pi. Then, a border detection algorithm is launched in the surface of interest at step S62.

The planes of the set of planes are successively processed according their position along the main axis. For example, they are processed for i starting from 1 up to 5 because along the main axis plane Pi+1 stands after plane Pi.

The algorithm draws the contour of the slice of the object in the current plane. Then, at step S63, the center of gravity of the contour is calculated.

This center of gravity serves as the "seed" for automatically selecting by the system the surface of interest at step S61 for the border detection algorithm in the next plane in the set of planes along the main axis.

This is achieved by geometrically projecting the center of gravity of a previously processed plane in a current plane along the main axis. This may also be achieved by using a common coordinates system and starting the algorithm at a point that has the same coordinates in the current plane as the center of gravity in the previous plane.

Indeed, as the planes of the set of planes are parallel one with respect to the others, by geometrical projection of the center of gravity of the contour in the next plane along the main axis, the "seed" for the border detection algorithm can be easily obtained.

At step T64, it is determined whether there remain other planes for contour detection. If there remain such planes, the position of the plane is incremented at step S65, and the reference plane is aligned with the next plane at step S66. Then, the process goes back to step S61

If there is no plane left in the set of planes for contour detection, all of the contours detected at step S62 are stacked along and perpendicularly to the main axis at step S67. And then, the volume of the object is calculated at step S68.

A device for implementing the above discussed method is described in view of figure 7. The device comprises an ultrasound probe 70 for emitting ultrasound waves towards an object 71 and receiving echoes of these waves reflected by the object. The signals delivered by the probe are processed by the acquisition module 72 for converting the signals into 3D image data. The device comprises a processor 73, for processing the image data according to the above discussed meshing or volume calculating method. The device also comprises a display unit for delivering to a monitor 75, images according to the reference plane. The device also comprises a communication module 77 for communicating with a computer 76. The device further comprises a mouse 78 for clicking on displayed images and selecting surfaces of interest.

The invention also relates to a computer program product that is able to implement any of the method steps as described above when loaded and run on computer means of an ultrasound imaging device. The computer program may be stored/distributed on a suitable medium supplied together with or as a part of other hardware, but may also be distributed in other forms, such as via the Internet or other wired or wireless telecommunication systems.

The invention also relates to an integrated circuit that is arranged to perform any of the method steps in accordance with the embodiments of the invention.

While the invention has been illustrated and described in detail in the drawings and foregoing description, such illustration and description are to be considered illustrative or exemplary and not restrictive, the invention being not restricted to the disclosed embodiment. Other variations to the disclosed embodiment can be understood and effected by those skilled in the art in practicing the claimed invention, from a study of the drawings, the disclosure and the appended claims.

In the claims, the word "comprising" does not exclude other elements or steps, and the indefinite article "a" or "an" does not exclude a plurality. A single processor or other unit may fulfil the functions of several items recited in the claims. The mere fact that different features are recited in mutually different dependent claims does not indicate that a combination of these features cannot be advantageously used. Any reference signs in the claims should not be construed as limiting the scope of the invention.

**Claims**

1. A method of automatically meshing a volume in an ultrasound imaging system, said method comprising the following steps:

   a) acquiring (S51) a set of 3D image data of an object;
   b) selecting by a user (S54) a first surface of interest in the 3D image data, said first surface of interest comprising a first slice of the object;
   c) automatically determining (S56) a main axis (AX) of the object in the first surface of interest;
   d) defining (S57) a first set of planes (22) of the 3D image data, these planes being not parallel with the main axis while being parallel one with respect to each other, with a given distance between two successive planes along the main axis ;

e) for at least two planes of the first set of planes, each comprising a respective second slice of the object, automatically drawing a contour (S62) of the second slice ;

f) automatically meshing the volume (S67) of the object, by stacking the contours drawn in said at least two planes of the first set of planes along the main axis and by separating these planes by said given distance.

2. The method according to claim 1, wherein step c) comprises the following sub-steps:

c1) applying a border detection algorithm in the first surface of interest for recognizing the first slice (CONT1); and

c2) selecting a segment in the recognized slice as the main axis.

3. The method according to claim 2, wherein the selected segment is the longest segment in the recognized slice.

4. The method according to any one of claims 2 to 3, further comprising the steps of:

- displaying at least an image of the first surface of interest;

- acquiring a user input indicating a region of the first surface of interest; and

- initiating the border detection algorithm in the region indicated by the user.

5. The method according to any one of the preceding claims, wherein step e) comprises the following sub-steps:

e1) calculating a center of gravity of a contour drawn in one plane of the first set of planes;

e2) selecting a second surface of interest in another plane of the first set of plane which is adjacent to said one plane along the main axis based on the calculated center of gravity; and

e3) triggering a border detection algorithm in said second surface of interest for drawing the contour in said another plane of the first set of planes.

6. The method according to any one of the preceding claims, further comprising the steps of :

- determining (S52) a second set of planes comprising at least two reference planes (MPR1, MPR2) in the set of 3D data, said reference planes being not parallel one with respect to each other;

- displaying 2D images of the set of 3D image data according to said reference planes;

- selecting one reference plane for the selection of the first surface of interest ins step b); and

- bringing (S66) to coincidence one plane of the set of planes with the other reference plane.

7. The method according to claim 6, wherein three reference planes are determined, each plane being perpendicular with respect to each other, and wherein a surface of interest for drawing the contour in step e) in at least one plane of the set of planes is designated by a point of intersection of the three reference planes.

8. A method of calculating a volume in an ultrasound system comprising the steps of :

- meshing the volume of the object by a method according to any one of claims 1 to 7, each plane of the set of planes being perpendicular to the main axis;

- calculating sub-volumes comprised between two successive planes in the set of planes with respect to the main axis; and

- summing (S68) said sub-volumes.

9. A computer program product comprising instructions for implementing the steps of a method according to any one of claims 1 to 7 when loaded and run on computer means of an ultrasound imaging device.

10. A computer program product comprising instructions for implementing the steps of a method according to claim 8 when loaded and run on computer means of an ultrasound imaging device.

11. A device for automatically meshing a volume of an object , said device comprising:

- means (70, 72) for acquiring a set of 3D image data by ultrasound;

- means (75, 74) for displaying at least an image of a slice of the object;

- means (73) for selecting by a user a first surface of interest in the 3D image data, said first surface of interest comprising a first slice of the object;

- means (73) for determining a main axis (AX) of the object in the surface of interest;

- means (73) for defining a first set of planes (22) of the 3D image data, these planes being not parallel with the main axis while being parallel one with respect to each other, with a given distance between two successive planes along the main axis;

- means (73) for drawing, in at least two planes of the first set of planes, each comprising a respective second slice of the object, a contour of the second slice; and

- means (73) for meshing the volume of the object by stacking the contours drawn in said at least two planes of the first set of planes along the main axis and separating the planes by said distance.

12. The device according to claim 11, further comprising means for automatically determining the main axis (AX) by applying a border detection algorithm in the first surface of interest for recognizing the first slice (CONT1), and means for selecting a segment in the recognized slice as the main axis.

13. The device according to any one of claims 12 through 13, further comprising :

    - means for displaying (75) at least an image comprising the first surface of interest;
    - means for acquiring a user input (78) indicating a region of the first surface of interest; and
    - means for initiating the border detection algorithm in the region indicated by the user.

14. The device according to any one of claims 11 to 13, further comprising:

    - means for calculating a center of gravity of a contour drawn in one plane of the first set of planes;
    - means for selecting a second surface of interest in another plane of the first set of plane which is adjacent to said one plane along the main axis based on the calculated center of gravity; and
    - means for triggering a border detection algorithm in said second surface of interest for drawing the contour in said another plane of the first set of planes.

15. The device according to any one of claims 11 to 14, further comprising:

    - means for determining a second set of planes comprising at least two reference planes (MPR1, MPR2) in the set of 3D data, said planes not being parallel one with the other;
    - means for displaying 2D images of the set of 3D image data according to said reference planes;
    - means for selecting one reference plane for the selection of the first surface of interest; and
    - means for bringing to coincidence one plane of the set of planes with the other reference plane.

**Patentansprüche**

1. Verfahren zur automatischen Vermaschung eines Volumens in einem Ultraschallbildgebungssystem, wobei das genannte Verfahren die folgenden Schritte umfasst:

    a) Erfassen (S51) eines Satzes dreidimensionaler Bilddaten eines Objekts;
    b) Auswählen einer ersten interessierenden Oberfläche in den dreidimensionalen Bilddaten durch den Benutzer, wobei die genannte erste interessierende Oberfläche eine erste Schicht des Objekts umfasst;
    c) automatisches Ermitteln (S56) einer Hauptachse (AX) des Objekts in der ersten interessierenden Oberfläche;
    d) Definieren (S57) einer ersten Gruppe von Ebenen (22) der dreidimensionalen Bilddaten, wobei diese Ebenen nicht parallel zu der Hauptachse liegen, jedoch parallel zueinander verlaufen, wobei zwischen zwei aufeinanderfolgenden Ebenen entlang der Hauptachse ein gegebener Abstand vorhanden ist;
    e) für mindestens zwei Ebenen der ersten Gruppe von Ebenen, die jeweils eine betreffende zweite Schicht des Objekts umfassen, automatisches Zeichnen einer Kontur (S62) der zweiten Schicht;
    f) automatisches Vermaschen des Volumens (S67) des Objekts durch Stapeln der in den genannten mindestens zwei Ebenen der ersten Gruppe von Ebenen gezeichneten Konturen entlang der Hauptachse und durch Trennen dieser Ebenen durch den genannten gegebenen Abstand.

2. Verfahren nach Anspruch 1, wobei Schritt c) die folgenden untergeordneten Schritte umfasst:

    c1) Anwenden eines Randdetektionsalgorithmus in der ersten interessierenden Oberfläche zum Erkennen der ersten Schicht (CONT1); und
    c2) Auswählen eines Segments in der erkannten Schicht als die Hauptachse.

3. Verfahren nach Anspruch 2, wobei das ausgewählte Segment das längste Segment in der erkannten Schicht ist.

4. Verfahren nach einem der Ansprüche 2 bis 3, das weiterhin die folgenden Schritte umfasst:

    - Anzeigen von mindestens einem Bild der ersten interessierenden Oberfläche;
    - Erfassen einer Benutzereingabe, die eine Region der ersten interessierenden Oberfläche angibt; und
    - Initiieren des Randdetektionsalgorithmus in der durch den Benutzer angegebenen Region.

**5.** Verfahren nach einem der vorhergehenden Ansprüche, wobei Schritt e) die folgenden untergeordneten Schritte umfasst:

e1) Berechnen eines Schwerpunkts einer in einer Ebene der ersten Gruppe von Ebenen gezeichneten Kontur;
e2) basierend auf dem berechneten Schwerpunkt Auswählen einer zweiten interessierenden Oberfläche in einer weiteren Ebene der ersten Gruppe von Ebenen, die der genannten einen Ebene entlang der Hauptachse benachbart ist; und
e3) Auslösen eines Randdetektionsalgorithmus in der genannten zweiten interessierenden Oberfläche zum Zeichnen der Kontur in der genannten weiteren Ebene der ersten Gruppe von Ebenen.

**6.** Verfahren nach einem der vorhergehenden Ansprüche, wobei das Verfahren weiterhin die folgenden Schritte umfasst:

- Ermitteln (S52) einer zweiten Gruppe von Ebenen mit mindestens zwei Bezugsebenen (MPR1, MPR2) in dem Satz der dreidimensionalen Daten, wobei die genannten Bezugsebenen nicht parallel zueinander verlaufen;
- Anzeigen von zweidimensionalen Bildern des Satzes von dreidimensionalen Bilddaten in Übereinstimmung mit den genannten Bezugsebenen;
- Auswählen einer Bezugsebene zur Auswahl der ersten interessierenden Oberfläche in Schritt b); und
- in-Deckung-Bringen (S66) einer Ebene der Gruppe von Ebenen mit der anderen Bezugsebene.

**7.** Verfahren nach Anspruch 6, wobei drei Bezugsebenen ermittelt werden, wobei die jeweiligen Ebene senkrecht in Bezug zueinander sind, und wobei eine interessierende Oberfläche zum Zeichnen der Kontur in Schritt e) in mindestens einer Ebene der Gruppe von Ebenen durch einen Schnittpunkt der drei Bezugsebenen bestimmt wird.

**8.** Verfahren zum Berechnen eines Volumens in einem Ultraschallsystem, wobei das Verfahren die folgenden Schritte umfasst:

- Vermaschen des Volumens des Objekts durch ein Verfahren nach einem der Ansprüche 1 bis 7, wobei jede Ebene der Gruppe von Ebenen senkrecht zu der Hauptachse verläuft;
- Berechnen von zwischen zwei aufeinanderfolgenden Ebenen in der Gruppe von Ebenen enthaltenen Teilvolumen in Bezug auf die Hauptachse; und
- Summieren (S68) der genannten Teilvolumen.

**9.** Computerprogrammprodukt mit Anweisungen zum Implementieren der Schritte eines Verfahrens nach einem der Ansprüche 1 bis 7, wenn es auf Computermitteln einer Ultraschallbildgebungsvorrichtung geladen und von diesen ausgeführt wird.

**10.** Computerprogrammprodukt mit Anweisungen zum Implementieren der Schritte eines Verfahrens nach Anspruch 8, wenn es auf Computermitteln einer Ultraschallbildgebungsvorrichtung geladen und von diesen ausgeführt wird.

**11.** Vorrichtung zur automatischen Vermaschung eines Volumens eines Objekts, wobei die genannte Vorrichtung Folgendes umfasst:

- Mittel (70, 72) zum Erfassen eines Satzes dreidimensionaler Bilddaten durch Ultraschall;
- Mittel (75, 74) zum Anzeigen mindestens eines Bilder von einer Schicht des Objekts;
- Mittel (73) zum Auswählen einer ersten interessierenden Oberfläche in den dreidimensionalen Bilddaten durch den Benutzer, wobei die genannte erste interessierende Oberfläche eine erste Schicht des Objekts umfasst;
- Mittel (73) zum Ermitteln einer Hauptachse (AX) des Objekts in der interessierenden Oberfläche;
- Mittel (73) zum Definieren einer ersten Gruppe von Ebenen (22) der dreidimensionalen Bilddaten, wobei diese Ebenen nicht parallel zu der Hauptachse liegen, jedoch parallel zueinander verlaufen, wobei zwischen zwei aufeinanderfolgenden Ebenen entlang der Hauptachse ein gegebener Abstand vorhanden ist;
- Mittel (73), um in mindestens zwei Ebenen der ersten Gruppe von Ebenen, die jeweils eine betreffende zweite Schicht des Objekts umfassen, eine Kontur der zweiten Schicht zu zeichnen; und
- Mittel (73) zum Vermaschen des Volumens des Objekts durch Stapeln der in den genannten mindestens zwei Ebenen der ersten Gruppe von Ebenen gezeichneten Konturen entlang der Hauptachse und durch Trennen der Ebenen durch den genannten Abstand.

**12.** Vorrichtung nach Anspruch 11, weiterhin mit Mitteln zum automatischen Ermitteln der Hauptachse (AX) durch Anwenden eines Randdetektionsalgorithmus in der ersten interessierenden Oberfläche zum Erkennen der ersten Schicht (CONT1), und mit Mitteln zum Auswählen eines Segments in der erkannten Schicht als die Hauptachse.

**13.** Vorrichtung nach einem der Ansprüche 12 bis 13, die weiterhin Folgendes umfasst:

- Mittel zum Anzeigen (75) von mindestens einem Bild, das die erste interessierende Oberfläche umfasst;
- Mittel zum Erfassen einer Benutzereingabe (78), die eine Region der ersten interessierenden Oberfläche angibt; und
- Mittel zum Initiieren des Randdetektionsalgorithmus in der durch den Benutzer angegebenen Region.

**14.** Vorrichtung nach einem der Ansprüche 11 bis 13, wobei die Vorrichtung weiterhin Folgendes umfasst:

- Mittel zum Berechnen eines Schwerpunkts einer in einer Ebene der ersten Gruppe von Ebenen gezeichneten Kontur;
- Mittel, um basierend auf dem berechneten Schwerpunkt eine zweiten interessierenden Oberfläche in einer weiteren Ebene der ersten Gruppe von Ebenen auszuwählen, die der genannten einen Ebene entlang der Hauptachse benachbart ist;
- Mittel zum Auslösen eines Randdetektionsalgorithmus in der genannten zweiten interessierenden Oberfläche zum Zeichnen der Kontur in der genannten weiteren Ebene der ersten Gruppe von Ebenen.

**15.** Vorrichtung nach einem der Ansprüche 11 bis 14, wobei die Vorrichtung weiterhin Folgendes umfasst:

- Mittel zum Ermitteln einer zweiten Gruppe von Ebenen mit mindestens zwei Bezugsebenen (MPR1, MPR2) in dem Satz der dreidimensionalen Daten, wobei die genannten Ebenen nicht parallel zueinander verlaufen;
- Mittel zum Anzeigen von zweidimensionalen Bildern des Satzes von dreidimensionalen Bilddaten in Übereinstimmung mit den genannten Bezugsebenen;
- Mittel zum Auswählen einer Bezugsebene zur Auswahl der ersten interessierenden Oberfläche; und
- Mittel, um eine Ebene der Gruppe von Ebenen mit der anderen Bezugsebene in Deckung zu bringen.

**Revendications**

**1.** Procédé de maillage automatique d'un volume dans un système d'imagerie à ultrasons, ledit procédé comprenant les étapes suivantes consistant à :

a) acquérir (S51) un ensemble de données d'image en 3D d'un objet ;
b) sélectionner par un utilisateur (S54) une première surface d'intérêt dans les données d'image en 3D, ladite première surface d'intérêt comprenant une première coupe de l'objet ;
c) déterminer automatiquement (S56) un axe principal (AX) de l'objet dans la première surface d'intérêt ;
d) définir (S57) un premier ensemble de plans (22) des données d'image en 3D, ces plans n'étant pas parallèles à l'axe principal tout en étant parallèles l'un à l'autre, avec une distance donnée entre deux plans successifs le long de l'axe principal ;
e) pour au moins deux plans du premier ensemble de plans, comprenant chacun une seconde coupe respective de l'objet, dessiner automatiquement un contour (S62) de la seconde coupe ;
f) mailler automatiquement les volumes (S67) de l'objet, en empilant les contours dessinés dans lesdits au moins deux plans du premier ensemble de plans le long de l'axe principal et en séparant ces plans de ladite distance donnée.

**2.** Procédé selon la revendication 1, dans lequel l'étape c) comprend les sous-étapes suivantes consistant à :

c1) appliquer un algorithme de détection de bordure dans la première surface d'intérêt pour reconnaître la première coupe (CONT1) ; et
c2) sélectionner un segment dans la coupe reconnue comme l'axe principal.

**3.** Procédé selon la revendication 2, dans lequel le segment sélectionné est le segment le plus long dans la coupe reconnue.

**4.** Procédé selon l'une quelconque des revendications 2 à 3, comprenant en outre les étapes consistant à :

- afficher au moins une image de la première surface d'intérêt ;
- acquérir une entrée utilisateur indiquant une région de la première surface d'intérêt ; et
- amorcer l'algorithme de détection de bordure dans la région indiquée par l'utilisateur.

**5.** Procédé selon l'une quelconque des revendications précédentes, dans lequel l'étape e) comprend les sous-étapes suivantes consistant à :

e1) calculer un centre de gravité d'un contour dessiné dans un plan du premier ensemble de plans ;
e2) sélectionner une seconde surface d'intérêt dans un autre plan du premier ensemble de plans qui est adjacent audit un plan le long de l'axe principal en se basant sur le centre de gravité calculé ; et

e3) déclencher un algorithme de détection de bordure dans ladite seconde surface d'intérêt pour dessiner le contour dans ledit autre plan du premier ensemble de plans.

**6.** Procédé selon l'une quelconque des revendications précédentes, comprenant en outre les étapes consistant à :

- déterminer (S52) un second ensemble de plans comprenant au moins deux plans de référence (MPR1, MPR2) dans l'ensemble de données en 3D, lesdits plans de référence n'étant pas parallèles l'un à l'autre ;
- afficher des images en 2D de l'ensemble de données d'image en 3D selon lesdits plans de référence ;
- sélectionner un plan de référence pour la sélection de la première surface d'intérêt dans l'étape b) ; et
- amener (S66) un plan de l'ensemble de plans en coïncidence avec l'autre plan de référence.

**7.** Procédé selon la revendication 6, dans lequel trois plans de référence sont déterminés, chaque plan étant perpendiculaire à l'autre, et dans lequel une surface d'intérêt permettant de dessiner le contour dans l'étape e) dans au moins un plan de l'ensemble de plans est désignée par un point d'intersection des trois plans de référence.

**8.** Procédé de calcul d'un volume dans un système à ultrasons comprenant les étapes consistant à :

- mailler le volume de l'objet par un procédé selon l'une quelconque des revendications 1 à 7, chaque plan de l'ensemble de plans étant perpendiculaires à l'axe principal ;
- calculer des sous-volumes compris entre deux plans successifs dans l'ensemble de plans par rapport à l'axe principal ; et
- sommer (S68) lesdits sous-volumes.

**9.** Produit de programme informatique comprenant des instructions permettant de mettre en oeuvre les étapes d'un procédé selon l'une quelconque des revendications 1 à 7 lorsqu'elles sont chargées et exécutées sur un moyen informatique d'un dispositif d'image à ultrasons.

**10.** Produit de programme informatique comprenant des instructions permettant de mettre en oeuvre les étapes d'un procédé selon la revendication 8 lorsqu'elles sont chargées et exécutées sur un moyen informatique d'un dispositif d'image à ultrasons.

**11.** Dispositif de maillage automatique d'un volume d'un objet, ledit dispositif comprenant :

- des moyens (70, 72) d'acquisition d'un ensemble de données d'image en 3D par ultrasons ;
- des moyens (75, 74) d'affichage d'au moins une image d'une coupe de l'objet ;
- un moyen (73) de sélection par un utilisateur d'une première surface d'intérêt dans les données d'image en 3D, ladite première surface d'intérêt comprenant une première coupe de l'objet ;
- un moyen (73) de détermination d'un axe principal (AX) de l'objet dans la surface d'intérêt;
- un moyen (73) de définition d'un premier ensemble de plans (22) des données d'image en 3D, ces plans n'étant pas parallèles à l'axe principal tout en étant parallèles l'un à l'autre, avec une distance donnée entre deux plans successifs le long de l'axe principal ;
- un moyen (73) de dessin, dans au moins deux plans du premier ensemble de plans, chacun comprenant une seconde coupe respective de l'objet, d'un contour de la seconde coupe ; et
- un moyen (73) de maillage du volume de l'objet en empilant les contours dessinés dans lesdits au moins deux plans du premier ensemble de plans le long de l'axe principal et en séparant les plans de ladite distance.

**12.** Dispositif selon la revendication 11, comprenant en outre un moyen de détermination automatique de l'axe principal (AX) en appliquant un algorithme de détection de bordure dans la première surface d'intérêt permettant de reconnaître la première coupe (CONT1), et un moyen de sélection d'un segment dans la coupe reconnue comme l'axe principal.

**13.** Dispositif selon l'une quelconque des revendications 11 à 12, comprenant en outre :

- un moyen d'affichage (75) d'au moins une image comprenant la première surface d'intérêt;
- un moyen d'acquisition d'une entrée utilisateur (78) indiquant une région de la première surface d'intérêt ; et
- un moyen d'amorçage de l'algorithme de détection de bordure dans la région indiquée par l'utilisateur.

**14.** Dispositif selon l'une quelconque des revendications 11 à 13, comprenant en outre :

- un moyen de calcul d'un centre de gravité d'un contour dessiné dans un plan du premier ensemble de plans ;

- un moyen de sélection d'une seconde surface d'intérêt dans un autre plan du premier ensemble de plans qui est adjacent audit un plan le long de l'axe principal en se basant sur le centre de gravité calculé ; et
- un moyen de déclenchement d'un algorithme de détection de bordure dans ladite seconde surface d'intérêt permettant de dessiner le contour dans ledit autre plan du premier ensemble de plans.

**15.** Dispositif selon l'une quelconque des revendications 11 à 14, comprenant en outre :

- un moyen de détermination d'un second ensemble de plans comprenant au moins deux plans de référence (MPR1, MPR2) dans l'ensemble de données en 3D, lesdits plans n'étant pas parallèles l'un à l'autre ;
- un moyen d'affichage d'images en 2D de l'ensemble de données d'image en 3D selon lesdits plans de référence ;
- un moyen de sélection d'un plan de référence pour la sélection de la première surface d'intérêt ; et
- un moyen permettant d'amener un plan de l'ensemble de plans en coïncidence avec l'autre plan de référence.

FIG.1.

FIG.7.

FIG.2.

FIG.3.

FIG.4.

```
┌──────────────┐
│    ACQUIS    │──S51
└──────────────┘
        │
┌──────────────┐
│   SET MPRS   │──S52
└──────────────┘
        │
┌──────────────┐
│  SLCT MPR1   │──S53
└──────────────┘
        │
┌──────────────┐
│     SOI      │──S54
└──────────────┘
        │
┌──────────────┐
│   DET CONT   │──S55
└──────────────┘
        │
┌──────────────┐
│    DET AX    │──S56
└──────────────┘
        │
┌──────────────┐
│    DEF Pᵢ    │──S57
└──────────────┘
        │
┌──────────────┐
│    i = 1     │──S58
└──────────────┘
        │
┌──────────────────┐
│ ALIGNMNT: MPR2=Pᵢ │──S59
└──────────────────┘
        │
   ( CONT DICTION )──S60
```

FIG.5.

FIG.6.

**EP 2 310 873 B1**

**Patent documents cited in the description**

- US 6106466 A